# FASCICULE DE BREVET EUROPEEN

(11) **EP 0 277 895 B1**
(45) Date de publication et mention de la délivrance du brevet: **13.07.1994**
(21) Numéro de dépôt: 88420025.4
(22) Date de dépôt: 28.01.1988
(51) Int. Cl.: A61K 39/395, A61K 9/20, A61K 9/18

(54) **Formes galéniques de gammaglobulines dites anti-inflammatoires pour administration par voie sub-linguale**
Galenische Formen von antiinflammatorischen Gammaglobulinen für die sublinguale Verabreichung
Galenical forms of antiinflammatory gammaglobulins for sublingual administration

(30) Priorité: 28.01.1987 FR 8701379
(43) Date de publication de la demande: 10.08.1988
(73) Titulaire: MEDIBREVEX SA, 74940 Annecy Le Vieux (FR)
(72) Inventeur: Bruttmann, Georges, F-38000 Grenoble (FR); Pedrali, Patrick, F-74000 Annecy Le Vieux (FR); Robert, Serge, Braine Le Chateau (BE)
(74) Mandataire: Maureau, Philippe

(56) Documents cités:
- EP-A- 0 094 575
- FR-A- 2 460 137
- G. NETIEN et al., "Galenica 16 - Médicaments homéopathiques", édition 2, 1986, Technique et Documentation, Paris (FR); pp. 77-99#

## Description

L'invention concerne des formes galéniques de gammaglobulines dites anti-inflammatoires pour administration par voie sub-linguale.

Deux types de préparation sont actuellement utilisées dans l'immunothérapie des affections inflammatoires rhumastismales et des affections auto-immunes.

Il s'agit d'une part des anti-IgG d'origine humaine qui inhibent, par rétro-action, la synthèse des immuno-globulines dirigées contre les antigènes HLA-DR (comme le facteur rhumatoïde par exemple).

Ces anti-IgG humaines (chaînes lourdes) sont obtenues à partir d'IgG humaines telles que celles provenant du Centre Français de Transfusion Sanguine (16,5 %) injectées chez le lapin et faisant l'objet d'un protocole d'obtention très rigoureux. On peut alors obtenir des solutions plasmatiques-mères qui contiennent, pour 100 ml, un gramme d'anticorps anti-IgG humaines.

L'autre type de préparation est un sérum de lapin anti-HLA obtenu à partir de lymphocytes dont tous les marqueurs HLA sauf un ont été couverts par l'immun-sérum correspondant. Les lymphocytes ainsi préparés n'ont donc plus qu'un seul déterminant HLA accessible. On peut donc dire que les sérums anti-HLA obtenus sont spécifiques, notamment B27, DR4.

Ces préparations sont actuellement appliquées par voie intra-dermique, et l'on connaît les problèmes que pose ce mode d'application douloureux et souvent suivi de réactions locales, voires générales ; ce mode d'application est donc mal accepté par le patient qui en vient souvent à refuser la poursuite du traitement.

Le document EP-A-0 094 575 décrit une forme galénique liquide d'administration par la voie sublinguale d'un mélange constitué par de l'histamine et une immunoglobuline anti-inflammatoire. L'histamine provoque la dilatation de différents tissus ou capillaires de l'organisme, et partant favorise le passage de l'immunoglobuline seule au travers de la muqueuse sublinguale. Au vu de ce document, rien ne permet d'indiquer ou de prévoir qu'une gammaglobuline puisse être administrée seule par la voie sublinguale. Et ce médicament requiert l'adjonction d'histamine dont les effets secondaires ne sont pas négligeables.

Il serait donc important de pouvoir disposer de nouvelles formes galéniques de gammaglobulines, spécifiques ou non spécifiques, qui ne présentent pas les inconvénients exposés ci-avant.

Les inventeurs ont, au cours de leurs études, réussi à mettre au point de nouvelles formes galéniques permettant l'application de ces gammaglobulines anti-inflammatoires par voie sub-linguale. On dispose ainsi d'un mode d'application totalement indolore, donc mieux toléré par le patient, ce qui permet, comme on le verra par la suite, d'élargir de façon importante les possibilités d'utilisation thérapeutique de ces produits.

Une composition immunothérapique selon l'invention, pour le traitement d'affections inflammatoires rhumatismales, se caractérise par le fait que les gamma-globulines anti-inflammatoires sont contenues, en quantités strictement contrôlées et reproductibles, dans un support solide pharmaceutiquement acceptable, adapté à une administration sublinguale.

Conformément au document suivant : "Galénica 16 - Médicaments homéopathiques, édition 2, 1986, pages 77-99, Technique et Documentation, Paris, France (G.NETIEN)", et pour des médicaments homéopathiques, on sait déjà obtenir des formes galéniques solides pour le support de différentes substances, aux fins d'une administration sublinguale. Mais en homéopathie, les différents principes actifs utilisés sont présents sur le support solide, en quantités infinitésimales, voire nulles, lesquelles en tous cas ne peuvent plus être dosées, et par conséquent contrôlées.

La préparation des nouvelles formes galéniques de gammaglobulines anti-inflammatoires selon l'invention va maintenant être décrite en détail, en commençant par la préparation des gammaglobulines selon les deux origines différentes ci-après.

### Préparation à partir d'anti-IgG humaines.

On sait que les immunoglobulines sont l'objet d'un mécanisme d'auto-régulation et que l'introduction d'une immunoglobuline à partir des cellules immuno-compétentes d'un mammifère conduit à inhiber la synthèse de l'immunoglobuline correspondante.

L'inhibition de synthèse d'une immunoglobuline nécessite donc que l'on dispose de cette immunoglobuline.

Dans le cas de l'immunothérapie d'affections inflammatoires rhumatismales, il est nécessaire, si l'on veut contrôler la synthèse des facteurs rhumatoïdes, de préparer cesdits facteurs rhumatoïdes afin de pouvoir introduire dans le corps humain les molécules correspondantes et de mettre en oeuvre le mécanisme d'auto-régulation.

Les facteurs rhumatoïdes sont des anti-immunoglobulines G, appartenant elles-mêmes aux trois principaux groupes IgM, IgA et IgG.

Leur préparation se fait donc à partir des IgG de l'homme, que l'on injecte au lapin selon le protocole suivant :
- IgG injectées : les IgG humaines à 16,5 % du Centre Français de Transfusion Sanguine
- Mode d'injection : intradermique en série de 25, d'un vingtième de ml (deux unités de la graduation "insuline").
- Adjuvant : produit MDP® de l'Institut Pasteur
- Nombre de séances : 3 à 15 jours d'intervalle.

L'animal est ensuite mis un mois au repos.

Le prélèvement sanguin est effectué sur le lapin par ponction artérielle au niveau de l'artère auriculaire médiane, après friction de l'oreille au xylol, avec une seringue de 10 ml. 1 ml d'héparinate de calcium est placé dans la seringue avant ponction. Les aiguilles sont de type "intradermique". La pression artérielle repousse le piston. La quantité de sang est de 5 ml.

Sous réserve d'un pansement compressif, pendant 5 minutes, à l'aide d'une boule de coton hydrophile, au point de prélèvement, l'animal pourra survivre à cette ponction, ce qui permet, le cas échéant, d'effectuer un second prélèvement du côté opposé.

Le sang hépariné est refoulé très doucement le long de la paroi d'un tube à centrifugation, puis centrifugé 5 minutes à 5 000 tours/minute.

Le plasma hépariné est recueilli et conserve aux environs de 0°C.

La réponse immune s'effectuant dans chacune des trois classes IgM, IgA et IgG, ce sont ces trois groupes d'anti-immunoglobulines qu'il faut récupérer dans le plasma hépariné total.

On peut, pour ce faire, extraire le fibrinogène et la sérumalbumine, puis utiliser le reliquat plasmatique.

Les inventeurs ont préféré utiliser une technique, dite extractive, c'est-à-dire susceptible de sélectionner, dans le plasma total, la fraction désirée et de la séparer des autres protéines. Cette méthode s'est, en effet révélée très supérieure quant à la qualité des produits fournis.

Cette technique d'extraction est la suivante :
Des hématies humaines, rincées, sans marqueurs de surface (0.-Rh-), sont tannées (acide tannique au 1/1 million, au 1/100.000) ; durée de contact 15 minutes à 37° ; rinçage au sérum physiologique ajusté à pH 7,2. La centrifugation ne doit jamais dépasser 450 tours/minute (risque d'agglutination irréversible des hématies tannées).

On place 1/10e de ml de ces hématies dans un tube à hémolyse, en suspension dans un tampon pH 6,4, dans lequel a été mis en suspension 1/10e de ml de la solution d'IgG à 16,5 % du CTS. (si possible provenant du même lot qui a servi à immuniser le lapin).

On laisse en contact dans un appareil à basculement lent (10 tours/minute), puis l'on sépare les hématies et on les rince. La centrifugation se fait à 450 tours/minute.

Les hématies tannées, sensibilisées, sont remises en suspension dans un tampon pH 7,2.

On met alors en contact le plasma hépariné du lapin et les hématies tannées sensibilisées.

1 ml de plasma hépariné est dilué dans 9ml de solution tampon pH 7,2.La suspension d'hématies a été étalonnée : 1/10 ml de culot pour 9,9 ml de tampon pH 7,2.

La mise en contact s'effectue dans un bécher, la solution et la suspension portées à 37°. Le bécher contenant les 2 parties est agité très doucement, puis après cinq minutes de contact, on centrifuge à 450 tours/minute pendant 5 minutes et le surnageant est enlevé.

Les hématies sont recueillies, placées dans un tube à centrifugation, rincées dans un tampon pH 7,2, essorées (450 tours/minute pendant 5 minutes). Le surnageant est aspiré et remplacé par un tampon pour élution (pH 3,2).

On laisse le tube bien bouché dans un appareil à basculement lent (10 tours/minute pendant 6 minutes).

Le surnageant récupéré contient les anti-IgG et constitue la solution de base, qui sera utilisée, comme on l'expliquera ci-après, pour la préparation des nouvelles formes galéniques.

La solution plasmatique mère contient pour 100 ml, 1 gramme d'anticorps anti-IgG humaines.

### Préparation du sérum de lapin anti-HLA.

L'animal est préparé par 4 séries de 20 injections intra-dermiques d'une suspension de lymphocytes dont tous les marqueurs HLA, sauf un, ont été couverts par l'immun-sérum correspondant. Les lymphocytes ainsi préparés n'ont plus qu'un déterminant HLA accessible.

Le sérum est prélevé un mois après la quatrième série d'injections. Il est absorbé sur les lymphocytes du même donneur et les immunoglobulines anti-HLA sont éluées sur un tampon pH 3,2. L'éluat contient donc tous les anticorps de la réponse immunitaire du lapin à l'injection des lymphocytes T de l'homme.

Pour séparer l'anticorps anti-HLA désiré (B27 dans 96 % des cas), on absorbe les anticorps anti-lymphocytes T, sur des lymphocytes B du même donneur qui ont été pré-traités avec le sérum anti-HLA correspondant et rincés à pH 7,2.

On recueille le surnageant, et après contrôles virologiques et bactériens, on le dilue au 1/100° pour obtenir la solution-mère, qui sera utilisée, comme on l'expliquera ci-après, pour la préparation des nouvelles formes galéniques selon l'invention.

Cette préparation des nouvelles formes galéniques pour administration par voie per- et sub-linguale va être décrite en détail.

On concentre sous vide les sérums ou solutions plasmatiques mères obtenus ci-avant, de façon à réduire le volume de liquide à imprégner. On peut également lyophiliser ces solutions.

On prépare ensuite des dilutions, en fonction des doses que l'on désire obtenir. Ces dilutions sont effectuées à l'aide de solvants convenables et notamment l'eau ou le sérum physiologique. Les doses actuellement préférées sont celles qui conduisent dans les formes galéniques à une teneur de 0,1 et 0,O1 mg d'anticorps anti-IgG.

On réalise ensuite, dans une turbine, à l'aide d'un injecteur du type dénommé "spray-doseur", l'imprégnation , à l'aide de chacune des dilutions ou sous-dilutions, de globules constitués, de façon connue en soi, d'un support solide pharmaceutiquement acceptable et spécialement d'un mélange saccharose-lactose. Il est bien évident que, sans sortir du cadre de l'invention, ces globules pourraient être remplacés par des poudres ou des comprimés, également adaptés à l'application par voie per-linguale ou sub-linguale.

La technique d'imprégnation est celle de la multi-imprégnation ou de l'imprégnation fractionnée, de manière à garantir une parfaite répartition du principe actif homogène.

Entre chaque imprégnation, le séchage est réalisé par passage d'air forcé et desséché dans une chambre où règne une dépression obtenue par la différence de débit existant entre ce passage d'air et un puissant extracteur.

Selon une caractéristique importante de l'invention, cette opération de séchage sera effectuée à une température inférieure ou égale à 30°C.

On a, en effet, déterminé, au cours des nombreux essais ayant conduit à la mise au point dudit procédé, que le procédé n'est plus fiable quand la température dépasse 38-40°C.

La dernière imprégnation est une imprégnation protectrice du type "dragéification".

Selon un autre mode de réalisation du procédé selon l'invention, et afin de garantir que soit totalement maintenue l'intégralité physicochimique du principe actif, les différentes étapes de l'imprégnation sont effectuées sous atmosphère d'azote.

La dernière opération consiste à mettre en gélules les globules ainsi obtenus, ou éventuellement à placer lesdits globules ou les poudres ou comprimés dans des tubes-doses et à terminer le conditionnement de façon classique (blisters pour les gélules, coffrets pour les tubes-doses) en numérotant, bien évidemment, les gélules à doses croissantes.

Ces préparations sont actuellement utilisées à des dosages de O,1 mg et O,O1 mg et permettent de traiter l'ensemble des pathologies, de type inflammatoire relevant du traitement de la classe thérapeutique des anti-inflammatoires stéroïdiens et non stéroïdiens.

La préparation anti-rhumatoïde-mère obtenue à partir des IgG humaines permet de traiter l'ensemble des rhumatismes inflammatoires, y compris la polyarthrite chronique rhumatismale.

La préparation obtenue à partir de sérum de lapin anti-HLA convient au traitement de la spondylarthrite ankylosante, et également à tout un ensemble d'arthrites.

Si on admet que les dysrégulations du système HLA peuvent être d'origine génétique ou non, il est logique de penser que les manifestations cliniques induites, articulaires par exemple, pourront être très rapides, violentes, très inflammatoires et très destructrices alors que la perte d'information est limitée a une longue suite de duplications cellulaires, il est évident que, par un processus différent, peuvent survenirbeaucoup plus tard, des atteintes de type auto-immun fragmentaires et dissociées et il s'agit d'arthrose. Ce deuxième type de produit donne des résultats remarquables également dans cette indication.

Les avantages des nouvelles formes galéniques de gammaglobulines anti-inflammatoires selon l'invention sont évidents, l'avantage principal étant que le patient n'est plus soumis aux aléas et aux inconvénients du traitement par injection.

D'autre part leurs procédés de fabrication sont simples et peu coûteux ; ils permettent d'obtenir des produits dosables, qu'il est possible de reproduire quantitativement et qualitativement, alors que jusqu'à ce jour, compte tenu du mode d'application par voie injectable intra-dermique, afin de stimuler les récepteurs portés sur les cellules LANGHERANS, les dilutions proposées étaient de type homéopathique.

Il est bien évident que la présente invention ne saurait se limiter aux concentrations, modes de fractionnement ou dilutions donnés ci-avant à titre d'exemple non limitatif ; ces concentrations, modes de fractionnement et dilutions peuvent, bien entendu, être adaptés sur demande ; de même, le support saccharose-lactose peut être remplacé par un autre support pharmaceutiquement acceptable adapté.

## Revendications (Revendications pour l'(les) Etat(s) contractant(s) suivant(s): AT, BE, CH, DE, FR, GB, IT, LI, LU, NL, SE)

1. Composition immunothérapique pour le traitement d'affections inflammatoires rhumatismales, comprenant des gammaglobulines anti-inflammatoires, **caractérisée en ce que** lesdites gammaglobulines sont contenues, en quantités strictement contrôlées et reproductibles, dans un support solide pharmaceutiquement acceptable, adapté à une administration sublinguale.

2. Composition selon la revendication 1, caractérisée en ce que les gammaglobulines sont sous forme lyophilisée dans le support solide.

3. Composition selon la revendication 1, caractérisée en ce que les gammaglobulines anti-inflammatoires sont obtenues à partir d'une solution plasmatique ou d'un sérum de lapin anti-HLA.

4. Composition selon la revendication 1, caractérisée en ce que les gammaglobulines anti-inflammatoires sont obtenues à partir d'une injection au lapin des IgG de l'homme, suivie d'une extraction du fibrinogène et de la sérumalbumine, et de la récupération du reliquat plasmatique.

5. Composition selon la revendication 1, caractérisée en ce que les gammaglobulines anti-inflammatoires sont obtenues à partir d'une injection au lapin des IgG de l'homme, suivie d'une extraction, dans le plasma total, de la fraction désirée, par mise en contact dudit plasma hépariné et d'hématies humaines, les autres protéines étant ensuite séparées.

6. Composition selon la revendication 1, caractérisée en ce que les gammaglobulines anti-inflammatoires sont obtenues à partir d'un sérum de lapin anti-HLA spécifique, obtenu à partir de lymphocytes dont tous les marqueurs HLA sauf un sont couverts par l'immunsérum correspondant.

7. Composition selon la revendication 1, caractérisée en ce que les gammaglobulines anti-inflammatoires sont des anti-immunoglobulines appartenant à l'un quelconque des groupes suivants, à savoir IgM, IgA et IgG.

## Revendications (Revendications pour l'(les) Etat(s) contractant(s) suivant(s): ES, GR)

1. Procédé de préparation d'une composition immunothérapique pour le traitement d'affections inflammatoires rhumatismales, à partir de gammaglobulines anti-inflammatoires, **caractérisé en ce qu'**on introduit lesdites gammaglobulines, en quantités strictement contrôlées et reproductibles, dans un support solide pharmaceutiquement acceptable, adapté à une administration sublinguale.

2. Procédé selon la revendication 1, caractérisé en ce que les gammaglobulines sont introduites sous forme lyophilisée dans le support solide.

3. Procédé selon la revendication 1, caractérisé en ce que les gammaglobulines anti-inflammatoires sont obtenues à partir d'une solution plasmatique ou d'un sérum de lapin anti-HLA.

4. Procédé selon la revendication 1, caractérisé en ce que les gammaglobulines anti-inflammatoires sont obtenues à partir d'une injection au lapin des IgG de l'homme, suivie d'une extraction du fibrinogène et de la sérumalbumine, et de la récupération du reliquat plasmatique.

5. Procédé selon la revendication 1, caractérisé en ce que les gammaglobulines anti-inflammatoires sont obtenues à partir d'une injection au lapin des IgG de l'homme, suivie d'une extraction, dans le plasma total, de la fraction désirée, par mise en contact dudit plasma hépariné et d'hématies humaines, les autres protéines étant ensuite séparées.

6. Procédé selon la revendication 1, caractérisé en ce que les gammaglobulines anti-inflammatoires sont obtenues à partir d'un sérum de lapin anti-HLA spécifique, obtenu à partir de lymphocytes dont tous les marqueurs HLA sauf un sont couverts par l'immun-sérum correspondant.

7. Procédé selon la revendication 1, caractérisé en ce que les gammaglobulines anti-inflammatoires sont des anti-immunoglobulines appartenant à l'un quelconque des groupes suivants, à savoir IgM, IgA et IgG.

## Claims (Claims for the following Contracting State(s): AT, BE, CH, DE, FR, GB, IT, LI, LU, NL, SE)

1. Immunotherapeutic composition for the treatment of inflammatory rheumatic disorders, comprising anti-inflammatory gamma globulins, characterized in that the said gamma globulins are contained, in strictly controlled and reproducible amounts, in a pharmaceutically acceptable solid carrier suitable for sublingual administration.

2. Composition according to Claim 1, characterized in that the gamma globulins are in lyophilized form in the solid carrier.

3. Composition according to Claim 1, characterized in that the anti-inflammatory gamma globulins are obtained from a rabbit anti-HLA plasma solution or serum.

4. Composition according to Claim 1, characterized in that the anti-inflammatory gamma globulins are obtained by means of an injection into rabbits of human IgG, followed by an extraction of the fibrinogen and the serum albumin and by recovery of the plasma remaining.

5. Composition according to Claim 1, characterized in that the anti-inflammatory gamma globulins are obtained by means of an injection into rabbits of human IgG, followed by an extraction of the desired fraction from within the total plasma by bringing the said heparinized plasma and human red cells into contact, the other proteins then being separated off.

6. Composition according to Claim 1, characterized in that the anti-inflammatory gamma globulins are obtained from a specific anti-HLA rabbit serum, obtained from lymphocytes all of whose HLA markers except one are covered with the corresponding immune serum.

7. Composition according to Claim 1, characterized in that the anti-inflammatory gamma globulins are anti-immunoglobulins belonging to any one of the following groups, namely IgM, IgA and IgG.

## Claims (Claims for the following Contracting State(s): ES, GR)

1. Process for preparing an immunotherapeutic composition for the treatment of inflammatory rheumatic disorders, using anti inflammatory gamma globulins, characterized in that the said gamma globulins are introduced, in strictly controlled and reproducible amounts, into a pharmaceutically acceptable solid carrier suitable for sublingual administration.

2. Process according to Claim 1, characterized in that the gamma globulins are introduced in lyophilized form into the solid carrier.

3. Process according to Claim 1, characterized in that the anti-inflammatory gamma globulins are obtained from a rabbit anti-HLA plasma solution or serum.

4. Process according to Claim 1, characterized in that the anti-inflammatory gamma globulins are obtained by means of an injection into rabbits of human IgG, followed by an extraction of the fibrinogen and the serum albumin and by recovery of the plasma remaining.

5. Process according to Claim 1, characterized in that the anti-inflammatory gamma globulins are obtained by means of an injection into rabbits of human IgG, followed by an extraction of the desired fraction from within the total plasma by bringing the said heparinized plasma and human red cells into contact, the other proteins then being separated off.

6. Process according to Claim 1, characterized in that the anti-inflammatory gamma globulins are obtained from a specific anti-HLA rabbit serum, obtained from lymphocytes all of whose HLA markers except one are covered with the corresponding immune serum.

7. Process according to Claim 1, characterized in that the anti-inflammatory gamma globulins are anti-immunoglobulins belonging to any one of the following groups, namely IgM, IgA and IgG.

## Patentansprüche (Patentansprüche für folgende(n) Vertragsstaat(en): AT, BE, CH, DE, FR, GB, IT, LI, LU, NL, SE)

1. Immunotherapeutische Zusammensetzung für die Behandlung von entzündlichen rheumatischen Erkrankungen, enthaltend entzündungshemmende Gammaglobuline, dadurch gekennzeichnet, daß die Gammaglobuline in genau kontrollierten und reproduzierbaren Mengen in einem pharmazeutisch akzeptablen festen Träger enthalten sind, der für eine sublinguale Verabreichung geeignet ist.

2. Zusammensetzung nach Anspruch 1, dadurch gekennzeichnet, daß die Gammaglobuline in lyophilisierter Form im festen Träger enthalten sind.

3. Zusammensetzung nach Anspruch 1, dadurch gekennzeichnet, daß die entzündungshemmenden Gammaglobuline aus einer plasmatischen Lösung oder einem Anti-HLH Kaninchenserum erhalten worden sind.

4. Zusammensetzung nach Anspruch 1, dadurch gekennzeichnet, daß die entzündungshemmenden Gammaglobuline aus einer Injektion von menschlichen IgG beim Kaninchen, gefolgt von einer Extraktion des Fibrinogens und des Serumalbumins, und einer Verwertung der plasmatischen Restmenge erhalten worden sind.

5. Zusammensetzung nach Anspruch 1, dadurch gekennzeichnet, daß die entzündungshemmenden Gammaglobuline aus einer Injektion von menschlichen IgG beim Kaninchen erhalten worden sind, gefolgt von einer Extraktion der gewünschten Fraktion aus dem Gesamtplasma, indem das heparinisierte Plasma und menschliche rote Blutkörperchen in Kontakt gebracht werden, wobei die anderen Proteine anschließend abgetrennt werden.

6. Zusammensetzung nach Anspruch 1, dadurch gekennzeichnet, daß die entzündungshemmenden Gammaglobuline aus einem spezifischen Anti-HLA Kaninchenserum erhalten worden sind, das aus Lymphozyten erhalten wurde, bei denen alle HLA-Marker, mit der Ausnahme von einem, durch das entsprechende Immunserum belegt sind.

7. Zusammensetzung nach Anspruch 1, dadurch gekennzeichnet, daß die entzündungshemmenden Gammaglobuline Anti-Immuno-Globuline sind, die einer der folgenden Gruppen, nämlich IgM, IgA und IgG zugehörig sind.

## Patentansprüche (Patentansprüche für folgende(n) Vertragsstaat(en): ES, GR)

1. Verfahren zum Herstellen einer immunotherapeutischen Zusammensetzung für die Behandlung von entzündlichen rheumatischen Erkrankungen, ausgehend von entzündungshemmenden Gammaglobulinen, dadurch gekennzeichnet, daß die Gammaglobuline in genau kontrollierten und reproduzierbaren Mengen in einem pharmazeutisch akzeptablen festen Träger eingebracht werden, der für eine sublinguale Verabreichung geeignet ist.

2. Verfahren nach Anspruch 1, dadurch gekennzeichnet, daß die Gammaglobuline in lyophilisierter Form in den festen Träger eingebracht werden.

3. Verfahren nach Anspruch 1, dadurch gekennzeichnet, daß die entzündungshemmenden Gammaglobuline aus einer plasmatischen Lösung oder einem Anti-HLA Kaninchenserum erhalten worden sind.

4. Verfahren nach Anspruch 1, dadurch gekennzeichnet, daß die entzündungshemmenden Gammaglobuline aus einer Injektion von menschlichen IgG beim Kaninchen, gefolgt von einer Extraktion des Fibrinogens und des Serumalbumins, und einer Verwertung der plasmatischen Restmenge erhalten worden sind.

5. Verfahren nach Anspruch 1, dadurch gekennzeichnet, daß die entzündungshemmenden Gammaglobuline aus einer Injektion von menschlichen IgG beim Kaninchen erhalten worden sind, gefolgt von einer Extraktion der gewünschten Fraktion aus dem Gesamtplasma, indem das heparinisierte Plasma und menschliche rote Blutkörperchen in Kontakt gebracht werden, wobei die anderen Proteine anschließend abgetrennt werden.

6. Verfahren nach Anspruch 1, dadurch gekennzeichnet, daß die entzündungshemmenden Gammaglobuline aus einem spezifischen Anti-HLA Kaninchenserum erhalten worden sind, das aus Lymphozyten erhalten wurde, bei denen alle HLA-Marker, mit der Ausnahme von einem, durch das entsprechende Immunserum belegt sind.

7. Verfahren nach Anspruch 1, dadurch gekennzeichnet, daß die entzündungshemmenden Gammaglobuline Anti-Immuno-Globuline sind, die einer der folgenden Gruppen, nämlich IgM, IgA und IgG zugehörig sind.
